# EUROPEAN PATENT APPLICATION

(11) **EP 2 856 869 A1**
(43) Date of publication of application: **08.04.2015**
(21) Application number: 13796611.5
(22) Date of filing: 30.05.2013
(51) Int. Cl.: A01K 67/027, C12N 15/09

(54) **AMYOTROPHIC LATERAL SCLEROSIS AND/OR FRONTOTEMPORAL LOBAR DEGENERATION MODEL MOUSE**

(30) Priority: 31.05.2012 JP 2012124947; 30.07.2012 JP 2012168001
(71) Applicant: Keio University, Tokyo 108-8345 (JP)
(72) Inventor: OKANO, Hideyuki, Tokyo 160-8582 (JP); OKANO, James Hirotaka, Tokyo 160-8582 (JP); MIYAUCHI, Chikako, Tokyo 160-8582 (JP)
(74) Representative: Creek, Isobel Clare
(86) International application number: PCT/JP2013/065029
(87) International publication number: WO 2013/180214

(57) **Abstract**

To generate a mouse model exhibiting symptoms similar to those of human amyotrophic lateral sclerosis and/or human frontotemporal lobar degeneration.

A mouse model exhibiting symptoms similar to those of human amyotrophic lateral sclerosis and/or human frontotemporal lobar degeneration can be produced by means of generating a mutation in a vertebrate so that expression of a mutant protein with a substitution of tyrosine for alanine at a position corresponding to position 382 of SEQ ID NO. 1 or a mutant protein with a substitution of cysteine for glycine at a position corresponding to position 348 of SEQ ID NO. 1 is regulated by an endogenous TDP-43 gene promoter in at least one allele of an endogenous TDP-43 gene of the vertebrate.

SEQUENCE LISTING

<110>  Keio University

<120>  ALS and/or FTLD model mouse

<130>  PCT2184

<160>  9

<170>  PatentIn version 3.5

<210>  1
<211>  414
<212>  PRT
<213>  Homo sapiens

<400>  1

<210>  2
<211>  414
<212>  PRT

<213>  Homo sapiens

<400>  2

Met Ser Glu Tyr Ile Arg Val Thr Glu Asp Glu Asn Asp Glu Pro Ile
1               5                   10                  15

Glu Ile Pro Ser Glu Asp Asp Gly Thr Val Leu Leu Ser Thr Val Thr
            20                  25                  30

Ala Gln Phe Pro Gly Ala Cys Gly Leu Arg Tyr Arg Asn Pro Val Ser
        35                  40                  45

Gln Cys Met Arg Gly Val Arg Leu Val Glu Gly Ile Leu His Ala Pro
    50                  55                  60

Asp Ala Gly Trp Gly Asn Leu Val Tyr Val Val Asn Tyr Pro Lys Asp
65                  70                  75                  80

Asn Lys Arg Lys Met Asp Glu Thr Asp Ala Ser Ser Ala Val Lys Val
                85                  90                  95

Lys Arg Ala Val Gln Lys Thr Ser Asp Leu Ile Val Leu Gly Leu Pro
            100                 105                 110

Trp Lys Thr Thr Glu Gln Asp Leu Lys Glu Tyr Phe Ser Thr Phe Gly
        115                 120                 125

Glu Val Leu Met Val Gln Val Lys Lys Asp Leu Lys Thr Gly His Ser
    130                 135                 140

Lys Gly Phe Gly Phe Val Arg Phe Thr Glu Tyr Glu Thr Gln Val Lys
145                 150                 155                 160

Val Met Ser Gln Arg His Met Ile Asp Gly Arg Trp Cys Asp Cys Lys
                165                 170                 175

Leu Pro Asn Ser Lys Gln Ser Gln Asp Glu Pro Leu Arg Ser Arg Lys
            180                 185                 190

Val Phe Val Gly Arg Cys Thr Glu Asp Met Thr Glu Asp Glu Leu Arg
        195                 200                 205

Glu Phe Phe Ser Gln Tyr Gly Asp Val Met Asp Val Phe Ile Pro Lys
    210                 215                 220

Pro Phe Arg Ala Phe Ala Phe Val Thr Phe Ala Asp Asp Gln Ile Ala
225                 230                 235                 240

Gln Ser Leu Cys Gly Glu Asp Leu Ile Ile Lys Gly Ile Ser Val His
                245                 250                 255

Ile Ser Asn Ala Glu Pro Lys His Asn Ser Asn Arg Gln Leu Glu Arg
            260                 265                 270

Ser Gly Arg Phe Gly Gly Asn Pro Gly Gly Phe Gly Asn Gln Gly Gly
        275                 280                 285

Phe Gly Asn Ser Arg Gly Gly Gly Ala Gly Leu Gly Asn Asn Gln Gly
    290                 295                 300

Ser Asn Met Gly Gly Gly Met Asn Phe Gly Ala Phe Ser Ile Asn Pro
305                 310                 315                 320

Ala Met Met Ala Ala Ala Gln Ala Ala Leu Gln Ser Ser Trp Gly Met
                325                 330                 335

Met Gly Met Leu Ala Ser Gln Gln Asn Gln Ser Gly Pro Ser Gly Asn
            340                 345                 350

Asn Gln Asn Gln Gly Asn Met Gln Arg Glu Pro Asn Gln Ala Phe Gly
        355                 360                 365

Ser Gly Asn Asn Ser Tyr Ser Gly Ser Asn Ser Gly Ala Thr Ile Gly
    370                 375                 380

Trp Gly Ser Ala Ser Asn Ala Gly Ser Gly Ser Gly Phe Asn Gly Gly
385                 390                 395                 400

Phe Gly Ser Ser Met Asp Ser Lys Ser Ser Gly Trp Gly Met
                405                 410

<210>  3
<211>  414
<212>  PRT
<213>  Homo sapiens

<400>  3

Met Ser Glu Tyr Ile Arg Val Thr Glu Asp Glu Asn Asp Glu Pro Ile
1               5                   10                  15

Glu Ile Pro Ser Glu Asp Asp Gly Thr Val Leu Leu Ser Thr Val Thr
            20                  25                  30

Ala Gln Phe Pro Gly Ala Cys Gly Leu Arg Tyr Arg Asn Pro Val Ser
        35                  40                  45

Gln Cys Met Arg Gly Val Arg Leu Val Glu Gly Ile Leu His Ala Pro
    50                  55                  60

Asp Ala Gly Trp Gly Asn Leu Val Tyr Val Val Asn Tyr Pro Lys Asp
65                  70                  75                  80

Asn Lys Arg Lys Met Asp Glu Thr Asp Ala Ser Ser Ala Val Lys Val
                85                  90                  95

Lys Arg Ala Val Gln Lys Thr Ser Asp Leu Ile Val Leu Gly Leu Pro
            100                 105                 110

Trp Lys Thr Thr Glu Gln Asp Leu Lys Glu Tyr Phe Ser Thr Phe Gly
        115                 120                 125

Glu Val Leu Met Val Gln Val Lys Lys Asp Leu Lys Thr Gly His Ser
    130                 135                 140

Lys Gly Phe Gly Phe Val Arg Phe Thr Glu Tyr Glu Thr Gln Val Lys
145                 150                 155                 160

Val Met Ser Gln Arg His Met Ile Asp Gly Arg Trp Cys Asp Cys Lys
                165                 170                 175

Leu Pro Asn Ser Lys Gln Ser Gln Asp Glu Pro Leu Arg Ser Arg Lys
            180                 185                 190

Val Phe Val Gly Arg Cys Thr Glu Asp Met Thr Glu Asp Glu Leu Arg
        195                 200                 205

Glu Phe Phe Ser Gln Tyr Gly Asp Val Met Asp Val Phe Ile Pro Lys
    210                 215                 220

Pro Phe Arg Ala Phe Ala Phe Val Thr Phe Ala Asp Asp Gln Ile Ala
225                 230                 235                 240

Gln Ser Leu Cys Gly Glu Asp Leu Ile Ile Lys Gly Ile Ser Val His
                245                 250                 255

Ile Ser Asn Ala Glu Pro Lys His Asn Ser Asn Arg Gln Leu Glu Arg
            260                 265                 270

Ser Gly Arg Phe Gly Gly Asn Pro Gly Gly Phe Gly Asn Gln Gly Gly
        275                 280                 285

Phe Gly Asn Ser Arg Gly Gly Gly Ala Gly Leu Gly Asn Asn Gln Gly
    290                 295                 300

Ser Asn Met Gly Gly Gly Met Asn Phe Gly Ala Phe Ser Ile Asn Pro
305                 310                 315                 320

Ala Met Met Ala Ala Ala Gln Ala Ala Leu Gln Ser Ser Trp Gly Met
                325                 330                 335

Met Gly Met Leu Ala Ser Gln Gln Asn Gln Ser Cys Pro Ser Gly Asn
            340                 345                 350

Asn Gln Asn Gln Gly Asn Met Gln Arg Glu Pro Asn Gln Ala Phe Gly
        355                 360                 365

Ser Gly Asn Asn Ser Tyr Ser Gly Ser Asn Ser Gly Ala Ala Ile Gly
    370                 375                 380

Trp Gly Ser Ala Ser Asn Ala Gly Ser Gly Ser Gly Phe Asn Gly Gly
385                 390                 395                 400

Phe Gly Ser Ser Met Asp Ser Lys Ser Ser Gly Trp Gly Met
                405                 410

<210>  4
<211>  27
<212>  DNA
<213>  Artificial Sequence

<220>
<223>  primer

<400>  4
attgtttccc aaactagctc caccccc                                          27

<210>  5
<211>  25
<212>  DNA
<213>  Artificial Sequence

<220>
<223>  primer

<400>  5
attaatgctg aacgtaccaa agttc                                            25

<210>  6
<211>  25
<212>  DNA
<213>  Artificial Sequence

<220>
<223>  primer

<400>  6

attacccgat gggcatgact ggttc 25

<210> 7
<211> 27
<212> DNA
<213> Artificial Sequence

<220>
<223> primer

<400> 7
ttggttttgg ttactacccg attggcc 27

<210> 8
<211> 27
<212> DNA
<213> Artificial Sequence

<220>
<223> primer

<400> 8
tccccaacca attgttgcac cagaatt 27

<210> 9
<211> 1440
<212> DNA
<213> Homo sapiens

<400> 9
ggtgggcggg gggaggaggc ggccctagcg ccattttgtg ggagcgaagc ggtggctggg 60

ctgcgcttgg gtccgtcgct gcttcggtgt ccctgtcggg cttcccagca gcggcctagc 120

gggaaaagta aaagatgtct gaatatattc gggtaaccga agatgagaac gatgagccca 180

ttgaaatacc atcggaagac gatgggacgg tgctgctctc cacggttaca gcccagtttc 240

caggggcgtg tgggcttcgc tacaggaatc cagtgtctca gtgtatgaga ggtgtccggc 300

tggtagaagg aattctgcat gccccagatg ctggctgggg aaatctggtg tatgttgtca 360

actatccaaa agataacaaa agaaaaatgg atgagacaga tgcttcatca gcagtgaaag 420

tgaaaagagc agtccagaaa acatccgatt taatagtgtt gggtctccca tggaaaacaa 480

ccgaacagga cctgaaagag tattttagta cctttggaga agttcttatg gtgcaggtca 540

agaaagatct taagactggt cattcaaagg ggtttggctt tgttcgtttt acggaatatg 600

aaacacaagt gaaagtaatg tcacagcgac atatgataga tggacgatgg tgtgactgca 660

aacttcctaa ttctaagcaa agccaagatg agcctttgag aagcagaaaa gtgtttgtgg 720

ggcgctgtac agaggacatg actgaggatg agctgcggga gttcttctct cagtacgggg 780

atgtgatgga tgtcttcatc cccaagccat tcagggcctt tgcctttgtt acatttgcag 840

atgatcagat tgcgcagtct ctttgtggag aggacttgat cattaaagga atcagcgttc 900

atatatccaa tgccgaacct aagcacaata gcaatagaca gttagaaaga agtggaagat 960

ttggtggtaa tccaggtggc tttgggaatc agggtggatt tggtaatagc agagggggtg 1020

gagctggttt gggaaacaat caaggtagta atatgggtgg tgggatgaac tttggtgcgt 1080

tcagcattaa tccagccatg atggctgccg cccaggcagc actacagagc agttggggta 1140

tgatgggcat gttagccagc cagcagaacc agtcaggccc atcgggtaat aaccaaaacc 1200

aaggcaacat gcagagggag ccaaaccagg ccttcggttc tggaaataac tcttatagtg 1260

gctctaattc tggtgcagca attggttggg gatcagcatc caatgcaggg tcgggcagtg 1320

gttttaatgg aggctttggc tcaagcatgg attctaagtc ttctggctgg ggaatgtaga 1380

cagtggggtt gtggttggtt ggtatagaat ggtgggaatt caaatttttc taaactcatg 1440

## Description

### Cross-reference to related applications

This application claims the benefit of Japanese Patent Application No. 2012-124947 filed on May 31, 2012 and Japanese Patent Application No. 2012-168001 filed on July 30, 2012, the entire disclosures of which are hereby incorporated by reference.

### Technical field

The present invention relates to a mouse model of amyotrophic lateral sclerosis and/or frontotemporal lobar degeneration.

### Background art

TAR DNA-binding protein 43kD (TDP-43) is a kind of heterogeneous nuclear ribonucleoproteins (hnRNPs). TDP-43 binds to mRNA or other hnRNP and is involved in, for example, stabilization of mRNA, alternative splicing of hnRNA, and transcriptional regulation. It was recently revealed that TDP-43 is a component of ubiquitin-positive inclusions found in affected regions of patients with frontotemporal lobar degeneration (FTLD) or amyotrophic lateral sclerosis (ALS) (M. Neumann et al. 2006 vol. 314 p. 130-133). Thereafter, a number of TDP-43 mutations were identified as a gene responsible for familial ALS (J. Sreedharan et al. 2008 vol. 319 p. 1668-1672; E. Lee wt al. 2012 vol. 13 p. 38-50).

Mutant TDP-43 transgenic mice and mutant TDP-43 (Q343R) knock-in mice were generated as TDP-43 mouse models of ALS, mimicking the mutations identified in human (FY2009 Annual Research Report of the Grants-in-Aid for Scientific Research, "Amyotrophic lateral sclerosis and TDP-43: elucidation of the entire neuropathological picture and molecular pathomechanism" (Research Project Number 20240037, Principal Investigator: TAKAHASHI, Hitoshi)). However, such neurological disorders as FTLD or ALS did not occur and abnormal intracellular inclusions leading to neuronal cell death were not detected. From these results, it was considered that other factor in addition to a mutation should be required for the development of a neurological disorder induced by a mutant TDP-43 (FY2009 Annual Research Report of the Grants-in-Aid for Scientific Research, "Amyotrophic lateral sclerosis and TDP-43: elucidation of the entire neuropathological picture and molecular pathomechanism" (Research Project Number 20240037, Principal Investigator: TAKAHASHI, Hitoshi)).

The present invention is directed to generate a mouse model with a disorder or disorders similar to human amyotrophic lateral sclerosis and/or human frontotemporal lobar degeneration.

### Summary of the invention

One aspect of the present invention is a non-human vertebrate in which expression of a mutant protein (SEQ ID NO. 2) with a substitution of tyrosine for alanine at a position corresponding to position 382 of SEQ ID NO. 1 or a mutant protein (SEQ ID NO. 3) with a substitution of cysteine for glycine at a position corresponding to position 348 of SEQ ID NO. 1 is regulated by an endogenous TDP-43 gene promoter in at least one allele of an endogenous TDP-43 gene. The vertebrate may have a mutation in which tyrosine is substituted for alanine at a position corresponding to position 382 of SEQ ID NO. 1 or a mutation in which cysteine is substituted for glycine at a position corresponding to position 348 of SEQ ID NO. 1, by an exogenous DNA, in at least one allele of the endogenous TDP-43 gene. The vertebrate may be an animal model of amyotrophic lateral sclerosis or frontotemporal lobar degeneration. It may be a knock-in mouse carrying a human TDP-43 gene.

Another aspect of the present invention is a method of producing an animal model of frontotemporal lobar degeneration or amyotrophic lateral sclerosis, comprising the step of generating a mutation into a non-human vertebrate so that expression of a mutant protein with a substitution of tyrosine for alanine at a position corresponding to position 382 of SEQ ID NO. 1 or a mutant protein with a substitution of cysteine for glycine at a position corresponding to position 348 of SEQ ID NO. 1 is regulated by an endogenous TDP-43 gene promoter in at least one allele of an endogenous TDP-43 gene of the vertebrate. In this method, the mutation may be generated in the vertebrate through the step of substituting tyrosine for alanine at a position corresponding to position 382 of SEQ ID NO. 1 or the step of substituting cysteine for glycine at a position corresponding to position 348 of SEQ ID NO. 1, using an exogenous DNA, in at least one allele of the endogenous TDP-43 gene of the vertebrate. In addition, in this method, the endogenous TDP-43 gene or a portion thereof, of the vertebrate may be substituted with the exogenous human TDP-43 gene or a portion thereof.

### Brief description of the drawings

Fig. 1 shows various images of abnormal inclusions that generated when a mutant TDP-43 (G348C) was transiently expressed in HeLa cells in one example of the present invention.
Fig. 2 shows time-series images of apoptosis that occurred when a mutant TDP-43 (G348C) was transiently expressed in HeLa cells in one example of the present invention.
Fig. 3 shows an image of abnormal inclusions that generated when a mutant TDP-43 (G348C) was transiently expressed in rat brain neurons in one example of the present invention, in which bright speckles are fluorescence signals from Venus and abnormal inclusions are observed in the cytoplasm of the neuron in the center.
Fig. 4 shows structures of a mutant TDP-43 knock-in vector (Targeting vector), an endogenous TDP-43 gene before knocking-in (Tardbp genome), and the endogenous TDP-43 gene after knocking-in, in one example of the present invention.
Fig. 5 shows expression of a wild-type or mutant TDP-43 in the central nervous system of wild-type and mutant TDP-43 knock-in mice in one example of the present invention. <G> represents a mutant TDP-43 (G348C) knock-in mouse and <A> represents a mutant TDP-43 (A382T) knock-in mouse.
Fig. 6 is a graph depicting changes in average body weights of wild-type and mutant TDP-43 knock-in mice recorded up to 14 months of age in one example of the present invention.
Fig. 7 represents images showing a behavioral abnormality due to defects of upper motor neurons in a mutant TDP-43 knock-in mouse in one example of the present invention.
Fig. 8 shows charts made by rating behavioral abnormalities (limb reflexes and tremor) of mutant TDP-43 knock-in mice in five grades and presenting the results for individual animals over time along with those of wild-type mice in one example of the present invention. <G> represents mutant TDP-43 (G348C) knock-in mice and <A> represents mutant TDP-43 (A382T) knock-in mice.
Fig. 9 shows charts generated by rating behavioral abnormalities (tremor, grip strength, and limb reflexes) of mutant TDP-43 knock-in mice and presenting the results for individual animals over time along with those of wild-type mice in one example of the present invention. <G> represents mutant TDP-43 (G348C) knock-in mice, <A> represents mutant TDP-43 (A382T) knock-in mice, and x represents that the behavioral abnormality could not be evaluated due to death of the subject.
Fig. 10 shows electromyograms obtained by measuring electromyographic activity of a mutant TDP-43 (G348C) knock-in mouse at 4 months of age in one example of the present invention. (A) electromyograms with prolonged MUP (Moter unit potential) durations; (B) an abnormal electromyogram; and (C) an electromyogram in which MUP duration was within a normal range.
Fig. 11 shows a fluorescence microscopic image of Venus in a brain section of a mutant TDP-43 knock-in mouse at 5 months of age in one example of the present invention. Arrows represent fluorescence signals from inclusions.
Fig. 12 shows changes in horizontal activity of a knock-in mouse (solid square) and a wild-type mouse (solid circle) in a home cage activity test in one example of the present invention.
Fig. 13 shows a graph of the time that a knock-in mouse (right bars) and a wild-type mouse (left bars) spent near the wall and the time that they spent in the central zone in an open field test in one example of the present invention.
Fig. 14 shows graphs of the time that knock-in mice (right bars) and wild-type mice (left bars) spent near a novel object in a novel object recognition test in one example of the present invention.
Fig. 15 shows graphs of the time that knock-in mice (right bars) and wild-type mice (left bars) spent in a chamber with a stranger mouse (stranger-near) and spent in a chamber without a stranger mouse (empty-near) in a three-chamber test in one example of the present invention.
Fig. 16 shows graphs of the time to consume all baits in the eight arms, the number of re-entries into a baited arm where the bait was already retrieved (the number of working memory errors), and the total number of entries into the arms for a knock-in mouse (solid square) and a wild-type mouse in a radial 8-arm maze test in one embodiment of the present invention.

### Embodiments for carrying out the invention

Unless otherwise noted in embodiments and examples, all procedures used are as described in standard protocols such as M. R. Green & J. Sambrook (Ed.), Molecular cloning, a laboratory manual (4th edition), Cold Spring Harbor Press, Cold Spring Harbor, New York (2012); F. M. Ausubel, R. Brent, R. E. Kingston, D. D. Moore, J.G. Seidman, J. A. Smith, K. Struhl (Ed.), Current Protocols in Molecular Biology, John Wiley & Sons Ltd., with or without modifications or changes. In addition, unless otherwise noted, a commercial reagent kit or a measurement instrument, if any, is used as described according to protocols attached thereto.

The above and further objects, features, advantages, and ideas of the present invention are apparent to those skilled in the art from consideration of the description of this specification. Furthermore, those skilled in the art can easily reproduce the present invention from the description herein. The mode(s) and specific example(s) described below represent a preferable embodiment of the present invention, which is given for the purpose of illustration or description. The present invention is not limited thereto. It is obvious to those skilled in the art that various changes and modifications may be made according to the description of the present specification without departing from the spirit and scope of the present invention disclosed herein.

### == Method of generating animal models of amyotrophic lateral sclerosis and/or frontotemporal lobar degeneration ==

Production of animal models with symptoms similar to those of amyotrophic lateral sclerosis and/or frontotemporal lobar degeneration comprises the step of generating a mutation in a vertebrate so that expression of a mutant protein with a substitution of tyrosine for alanine at a position corresponding to position 382 of SEQ ID NO. 1 or a mutant protein with a substitution of cysteine for glycine at a position corresponding to position 348 of SEQ ID NO. 1 is regulated by the endogenous TDP-43 gene promoter in at least one allele of the endogenous TDP-43 gene of a vertebrate.

TDP-43 gene has been identified in, for example, Homo sapiens (Gene ID: 23435), Mus musculus (Gene ID: 230908), and Drosophila melanogaster (Gene ID: 37781) and widely present in vertebrates. The vertebrate used is thus not specifically limited but is preferably a non-human primate or a rodent. A marmoset, a mouse, or a rat is particularly preferred. In at least one allele of the endogenous TDP-43 gene of the vertebrate, either the amino acid at the position corresponding to position 382 of SEQ ID NO. 1 is alanine or the amino acid at the position corresponding to position 348 of SEQ ID NO. 1 is glycine. It should be noted that the amino acid at the position corresponding to position 382 of SEQ ID NO. 1 does not necessarily present at position 382 in a given vertebrate, but may present at the position corresponding to position 382 of SEQ ID NO. 1, i.e., may be the amino acid at the position corresponding to position 382 of SEQ ID NO. 1 in an amino acid sequence homologous to the amino acid sequence around position 382 of SEQ ID NO. 1). The same applies to the amino acid at the position corresponding to position 348 of SEQ ID NO. 1.

An animal model of amyotrophic lateral sclerosis and/or frontotemporal lobar degeneration may be produced, for example, by using forward genetics in which a vertebrate is exposed to a mutagen and mutants with a substitution of tyrosine for the alanine or those with a substitution of cysteine for the glycine are selected from a number of mutants. Alternatively, reverse genetics may be used to substitute tyrosine for the alanine or substitute cysteine for the glycine with an exogenous DNA. For the amino acid substitution, the resulting mutant may also have, for example, other insertion of an exogenous DNA as long as expression of the mutant protein is regulated by the endogenous TDP-43 gene promoter and the effect of the mutant protein is exerted. Exemplified methods of amino acid substitution are given below, but the method of substitution is not limited to these examples.

First, alanine at a position corresponding to position 382 of SEQ ID NO. 1 is substituted with tyrosine or glycine at the position corresponding to position 348 of SEQ ID NO. 1 is substituted with cysteine, in an isolated TDP-43 gene or cDNA, using known in vitro mutagenesis. If necessary, DNA encoding a tag is ligated in-frame to the mutated gene or cDNA so that a fusion protein of the mutated TDP-43 and the tag can be expressed. Alternatively, an IRES sequence (internal ribosomal entry site) may be inserted between the mutated TDP-43 gene or cDNA and a gene encoding a tag so that two proteins can be expressed separately.

The origin of the TDP-43 gene or cDNA is not specifically limited, but a vertebrate TDP-43 gene or cDNA is preferable and a human TDP-43 gene or cDNA is more preferable. The tag is also not specifically limited, and examples thereof include oligopeptides such as a His tag and a Myc tag, fluorescent proteins such as GFP and EGFP, and enzymes such as beta-galactosidase, luciferase, and alkaline phosphatase. For fusion proteins, an oligopeptide or a fluorescent protein is preferred. If the IRES sequence is used, a fluorescent protein or an enzyme protein is preferred.

The generated knock-in DNA is introduced into pluripotent stem cells such as ES cells or iPS cells, and the cells in which the endogenous TDP-43 gene is replaced by the knock-in DNA are then selected. In the resulting cells, expression of the mutant protein with the substitution of tyrosine for alanine at a position corresponding to position 382 of SEQ ID NO. 1 or the mutant protein with the substitution of cysteine for glycine at a position corresponding to position 348 of SEQ ID NO. 1 is regulated by the endogenous TDP-43 gene promoter in one of the alleles of the endogenous TDP-43 gene.

Thereafter, chimeric animals are made using the cells thus obtained, and offspring in which the mutated TDP-43 gene is knocked in can be obtained from the chimeric animals having the pluripotent stem cells in their germ line. Alternatively, knock-in animals may be made by injecting the knock-in DNA into fertilized eggs, selecting individuals in which the endogenous TDP-43 gene is replaced, and growing the selected individuals into adults. The resulting knock-in animals are heterozygous for the mutant TDP-43 gene, but these heterozygotes can be used as animal models of amyotrophic lateral sclerosis and/or frontotemporal lobar degeneration because the introduced mutation is a dominant mutation. Homozygotes can be obtained by mating heterozygous male and female animals.

### == Animal model of amyotrophic lateral sclerosis and/or frontotemporal lobar degeneration ==

In vertebrates generated using the method described above, expression of the mutant protein with the substitution of tyrosine for alanine at a position corresponding to position 382 of SEQ ID NO. 1 or the mutant protein with the substitution of cysteine for glycine at a position corresponding to position 348 of SEQ ID NO. 1 is regulated by the endogenous TDP-43 gene promoter in at least one allele of the endogenous TDP-43 gene. The mutant vertebrates may have a mutation in which tyrosine is substituted for alanine at a position corresponding to position 382 of SEQ ID NO. 1 or a mutation in which cysteine is substituted for glycine at a position corresponding to position 348 of SEQ ID NO. 1, by an exogenous DNA, in at least one allele of the endogenous TDP-43 gene. It should be noted that the mutant vertebrates may also have, for example, other insertion of an exogenous DNA as long as expression of the mutant protein is regulated by the endogenous TDP-43 gene promoter and the effect of the mutant protein is exerted.

Since these mutations are dominant, the mutant vertebrates may be heterozygotes in which one allele of the endogenous TDP-43 gene is replaced. Alternatively, they may be homozygotes in which both alleles are replaced.

It is preferable that the endogenous TDP-43 gene is replaced by the exogenous human TDP-43 gene.

The mutant vertebrates exhibit, after birth, abnormal neurological disorders such as tremor (shivering) or abnormal reflexes of the limbs. In addition, the mutant vertebrates show a reduction in grip strength, and have trouble in gripping objects. Abnormal TDP-43 intracellular inclusions are formed in neurons in the central nervous system such as the spinal cord and the brain. Thus, since the vertebrates show symptoms specific to amyotrophic lateral sclerosis, they are useful as an animal model of amyotrophic lateral sclerosis.

On the other hand, in pathological observations of human FTLD, abnormal TDP-43 intracellular inclusions are also found in the brain. This pathological factor is considered to lead to reduction and loss of neurons in the frontal and temporal lobes as well as cerebral atrophy in the brain (frontal and temporal lobes) which can be examined by an imaging technique such as MRI. Thus, since the vertebrates show a symptom specific to frontotemporal lobar degeneration, namely, accumulation of abnormal TDP-43 intracellular inclusions, indicating ongoing degeneration of the neurons, they are useful as an animal model of frontotemporal lobar degeneration.

### Examples

### (1) Vector construction

Mutant TDP-43 cDNAs (G298S, A315T, G348C, N352S, and A382T) were generated by in vitro mutagenesis (Nuc. Acids Res. 2000 vol. 28 E78) using a plasmid with TDP-43 cDNA (Human cDNA clone, FCC-101, TOYOBO Co., LTD.)(SEQ ID NO. 9) inserted into HindIII and BamHI sites of a pBluescript SK(+) plasmid vector, and following primers.
G298S primer: attgtttcccaaactagctccaccccc (SEQ ID NO. 4)
A315T primer: attaatgctgaacgtaccaaagttc (SEQ ID NO. 5)
G348C primer: attacccgatgggcatgactggttc (SEQ ID NO. 6)
N352S primer: ttggttttggttactacccgattggcc (SEQ ID NO. 7)
A382T primer: tccccaaccaattgttgcaccagaatt (SEQ ID NO. 8)

Next, the mutant TDP-43 cDNAs cleaved with HindIII and KpnI were inserted into HindIII and KpnI sites of Venus/pcDNA3 that is a plasmid in which Venus is inserted into BamHI and EcoRI sites of the pcDNA3 vector. In this way, expression vectors were constructed in which the fluorescent protein Venus is fused to the mutant TDP-43.

### (2) Inclusion formation

The mutant TDP-43 expression vectors were lipofected into HeLa cells with Lipofectamin 2000, and the fluorescence of Venus was observed using a confocal fluorescence microscope after 24 hours. The frequency of cells with cytoplasmic inclusions containing Venus was the highest in the mutant TDP-43 (G348C) and the mutant TDP-43 (A382T), whereas only few inclusions were found by forced expression of wild-type TDP-43. These mutants were thus used to generate mutant TDP-43 knock-in mice. As similar results were obtained for the mutant TDP-43 (G348C) and the mutant TDP-43 (A382T) in all of the following experiments, those obtained for the mutant TDP-43 (G348C) are described herein as examples.

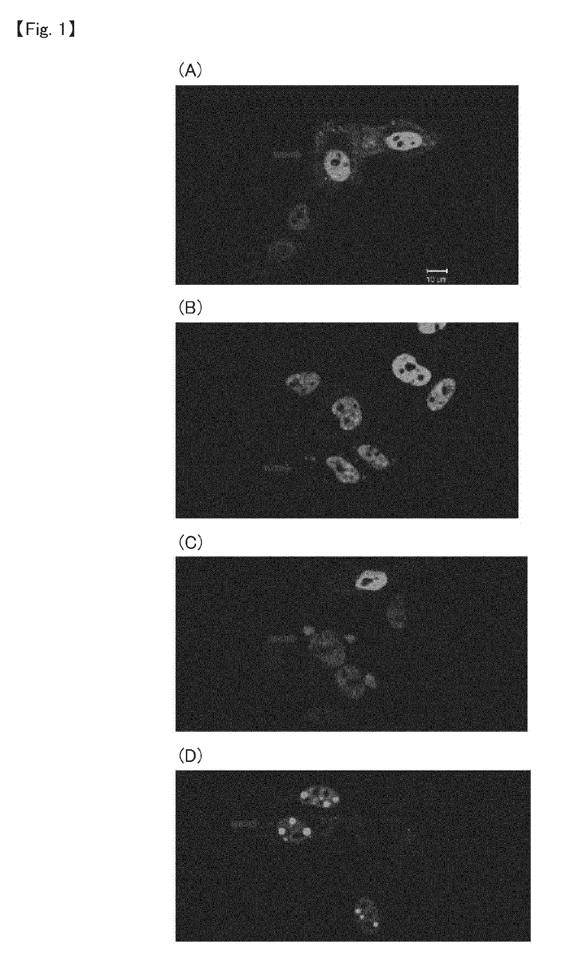

Fig. 1 shows various inclusions found for the mutant TDP-43 in the HeLa cells. Figs. 1A to 1C show examples of inclusions developed in cytoplasm: Fig. 1A shows relatively small inclusions that are diffused; Fig. 1B shows inclusions as medium-sized aggregates; and Fig. 1C shows inclusions as relatively large aggregates. Fig. 1D shows inclusions developed in nucleus.

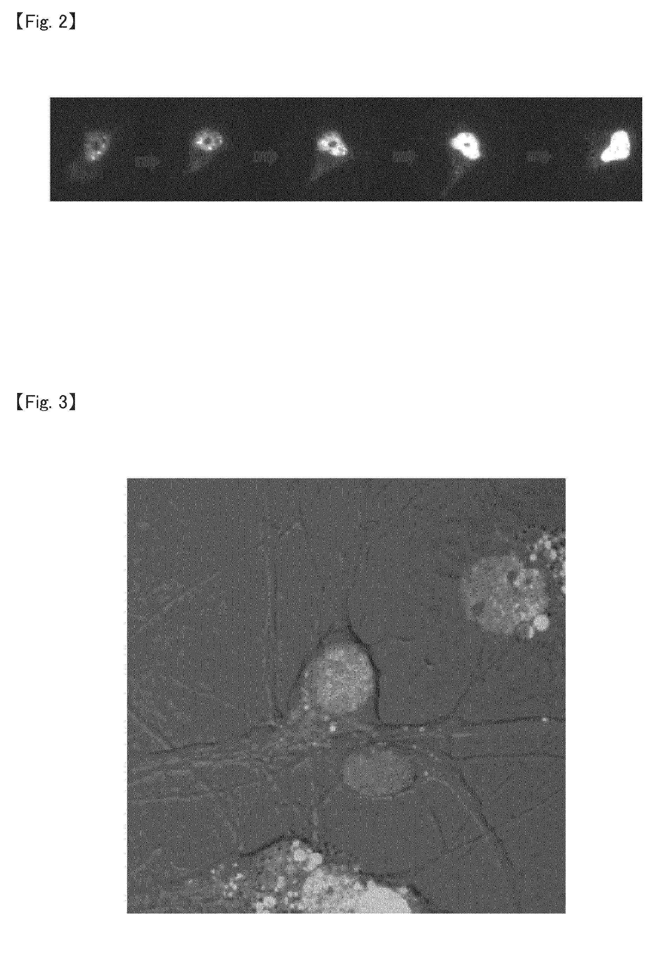

Next, cell death of the HeLa cells was detected using live cell imaging with bright-field and fluorescent microscopy. More specifically, as shown in Fig. 2, as the mutant TDP-43 leaks from the nucleus to the cytoplasm and forms inclusions over time, the cells became distorted. Fluorescence representing expression of the mutant TDP-43 was diminished at the timing of the cell death observed under bright field illumination. This indicates that the formation of the abnormal intracellular inclusions is the cause of the cell death.

Next, an effect of the mutant TDP-43 was observed in a similar manner using rat brain neurons. First, a Wistar rat at 19 days of pregnancy was sacrificed by cervical dislocation under anesthesia and its abdominal cavity was opened to remove a fetus. The brain was removed from the fetus and the hippocampus was dissected out under a stereomicroscope. The hippocampus obtained was immersed in PBS containing enzymes (Papain and DnaseI) at 37°C for 10 minutes. Cells from hippocampal tissue were dispersed with a glass pipette. The cells derived from the hippocampus were cultured on a 35-mm imaging dish. The medium used was 2% FBS/MEM supplemented with N2 and B27. On day 4 of cultivation, the mutant TDP-43 expression vector was transfected into the cultured cells of the rat hippocampus by calcium phosphate transfection. As a result, the formation of abnormal intracellular inclusions was again observed as shown in Fig. 3.

### (3) Generation of mutant TDP-43 knock-in mice

First, a vector with a Neo cassette inserted between XhoI and HindIII of a pBluescript vector was digested with ApaI and XhoI, into which mutant TDP-Venus-polyA excised out with ApaI and XhoI from the mutant TDP-43 expression vector was inserted to prepare TDP43-Venus-polyA-Neo cassette/pBstSK.

DNA fragment amplified by PCR using this vector as a template and a BAC (bacterial artificial chromosome) vector with an insertion of a wild-type TDP-43 gene were transformed into Ecoli by electroporation to induce homologous recombination between them to generate a knock-in vector.

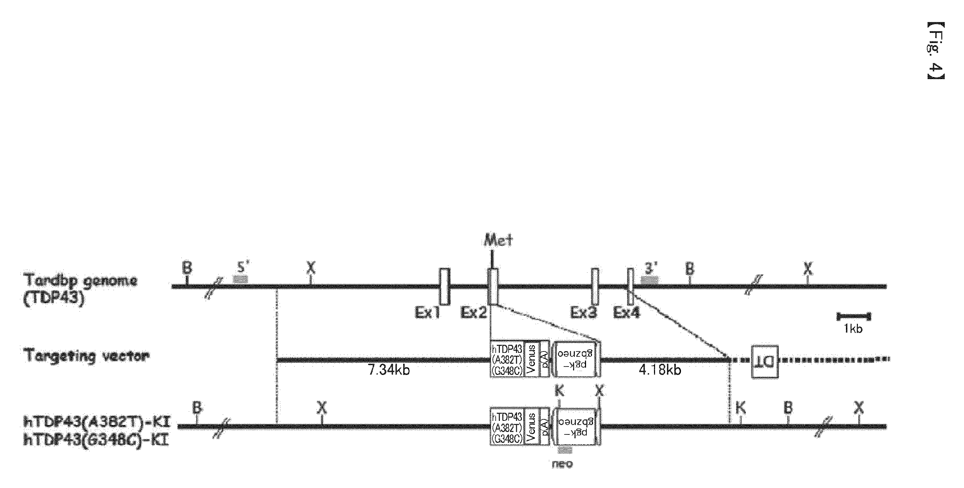

This vector was electroporated into the ES cells, and clones obtained by selection with G418 were screened for homologous recombinant ES cells. In this way, selected were clones in which the exon 2 of the endogenous TDP-43 gene was replaced with a portion of the mutant TDP-43 protein extending from the starting ATG, i.e., initiating methionine, to the HindIII site. Fig. 4 shows the structure of the knock-in vector, the endogenous TDP-43 gene before knocking-in, and the endogenous TDP-43 gene after knocking-in.

Chimeric mice were then generated using the ES cells, and germ-line chimeric mice were selected. Mutant TDP-43 knock-in mice were obtained from among their offspring.

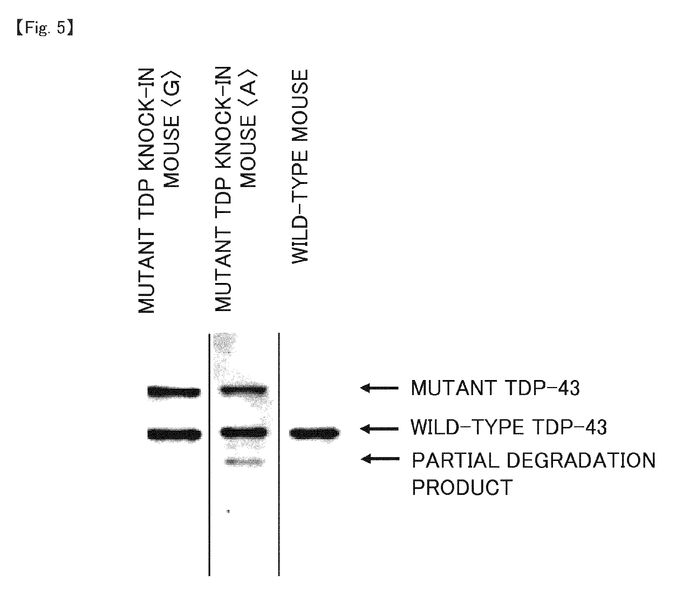

Western blotting was performed to determine expression of the knocked-in mutant TDP-43 in the mutant TDP-43 knock-in mice. More specifically, brains were removed from wild-type and mutant TDP-43 knock-in mice at 4 months of age. Crude brain extracts were then prepared and proteins were separated on an SDS-PAGE. The proteins were transferred to a PVDF membrane. Signals of the mutant TDP-43 were obtained using an anti-TARDBP polyclonal antibody (Proteintech, Catalog number: 12892-1-AP). The result is given in Fig. 5.

For the wild-type mouse, a signal for the wild-type TDP-43 was detected. For the mutant TDP-43 (A382T) knock-in mouse, signals representing the expression of the wild-type TDP-43 and mutant TDP-43 were detected. The level of expression of the wild-type TDP-43 in the wild-type animal was almost identical to the total level of expression of the wild-type TDP-43 and the mutant TDP-43 in the mutant mouse. In the mutant TDP-43 (A382T) knock-in mouse, in addition to these signals, a weak signal at a lower molecular weight was also detected. It is, however, generally considered that mutant proteins are susceptible to degradation. Accordingly, this extra signal is thought to be due to partial degradation of the mutant TDP-43 (A382T).

In the mutant TDP-43 (A382T) knock-in mice, it was thus confirmed that the knocked-in mutant gene was under the regulation similar to that in the wild-type mice.

### (4) Measurement of body weights of mutant TDP-43 knock-in mice

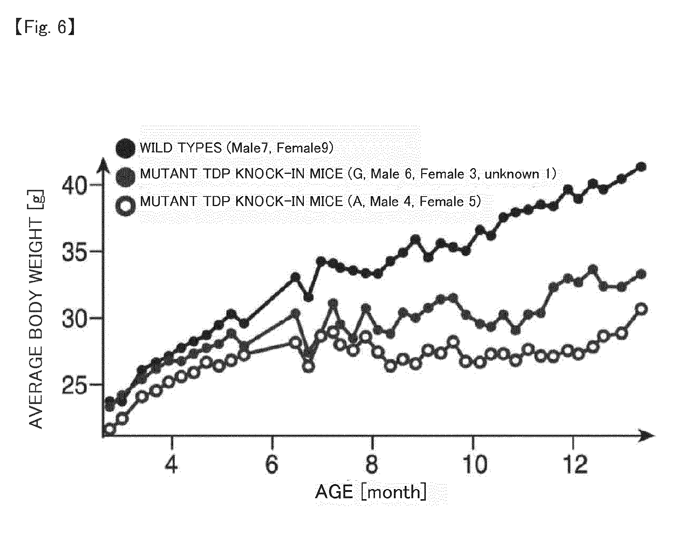

Wild-type mice (7 males and 9 females), mutant TDP-43 (G348C) knock-in mice (6 males and 3 females), and mutant TDP-43 (A382T) knock-in mice (4 males and 5 females) were weighed from immediately after birth to 14 months of age. Their average body weights were calculated and changes in the average body weights were plotted (Fig. 6).

As shown in Fig. 6, both types of the knock-in mice showed a slower increase in body weight relative to the wild-type, and were significantly lighter than the wild-type mice even after 14 months.

### (5) Behavior analysis of mutant TDP-43 knock-in mice

Behavior of the wild-type mice and the mutant TDP-43 knock-in mice was observed in a cage. The wild-type could grip the wire grid of the cage and move freely in the cage, whereas the both types of the mutant TDP-43 knock-in mice showed a reduction of grip strength. These animals could not support their own weight even when they grip the wire grid of the cage, and thus they were sluggish in their movements. This abnormality is thought to be due to defects of lower motor neurons.

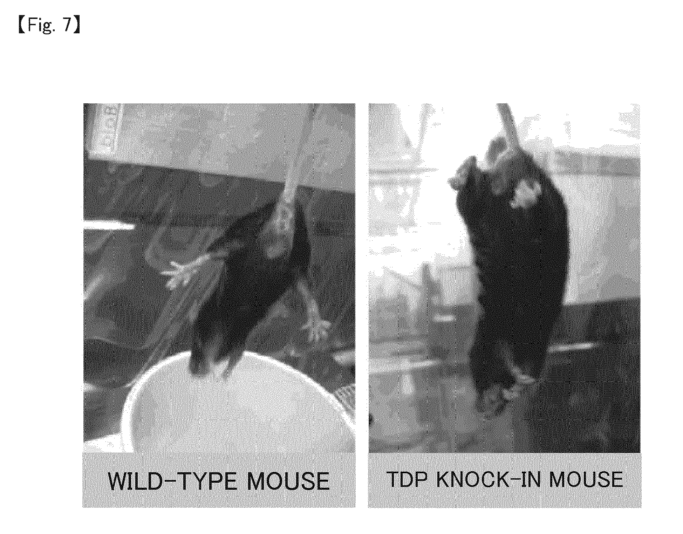

When mice are lifted by their tail, the normal mice extend their hind limbs straight out to keep the balance of the body, whereas both types of the abnormal mice cannot keep their balance with hind limb clasping (Fig. 7). This abnormality was not observed in the 16 wild-type mice, but started to appear at around the age of 6 months in the mutant mice: 6 out of 10 mutant TDP-43 (G348C) knock-in mice and 6 out of 9 mutant TDP-43 (A382T) knock-in mice continued to show the abnormality. This abnormality is thought to be due to defects of upper motor neurons.

Furthermore, mice moving freely in the cage were observed for 1-2 minutes to determine whether the tremor occurs. No tremor was observed in the 16 wild-type mice. In the mutant mice, however, tremor was continuously present in 8 out of 10 mutant TDP-43 (G348C) knock-in mice and 7 out of 9 mutant TDP-43 (A382T) knock-in mice.

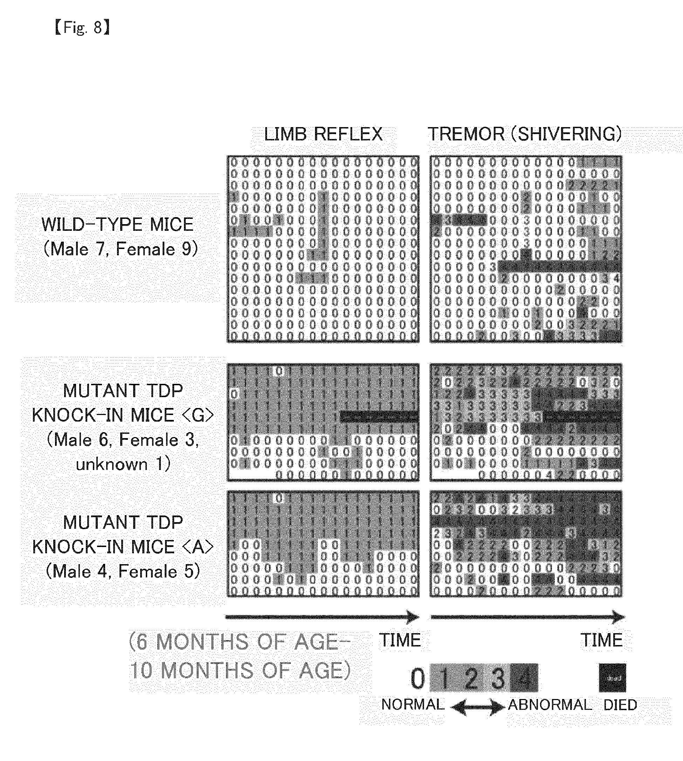

In order to quantify these behavioral abnormalities, limb reflexes and tremors were scored, and rated in five grades (0 to 4: the number of limbs showing abnormal reflex or tremor was used as scores) every week from 6 to 10 months of age. The results were presented as score charts for individual animals over time (Fig. 8). It can be seen from Fig. 8 that the tremors in the mutant mice were getting worse gradually between 6 and 10 months of age. For the limb reflexes, abnormalities were already found in many mutant mice at the time of 6 months of age.

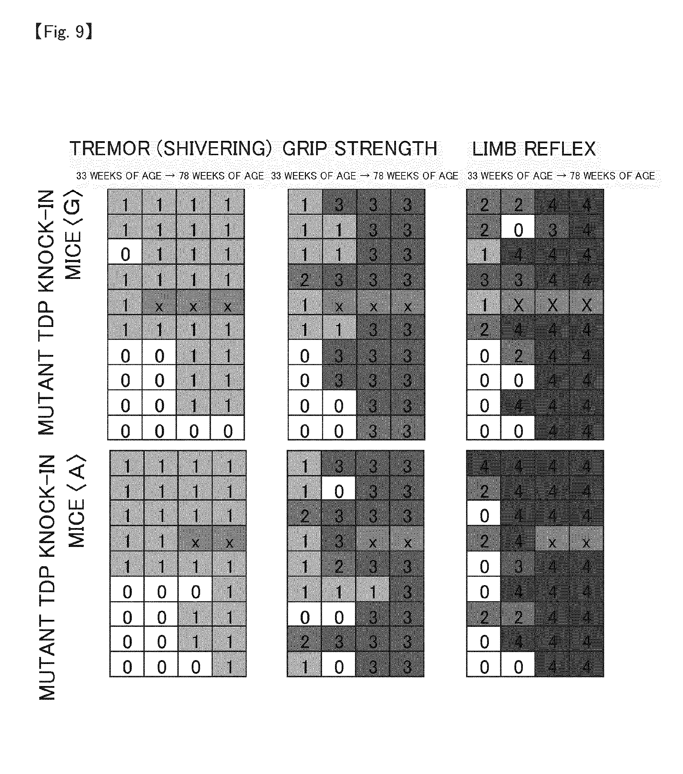

Furthermore, the observation period was extended and tremor, grip strength and limb reflexes were evaluated every 15 weeks from 33 to 78 weeks of age in the mutant TDP-43 (G348C) and TDP-43 (A382T) knock-in mice. For the tremor, the score was "0" if tremor was observed, and the score was "1" if no tremor was observed. For the grip strength, the score was "0" if the mouse was able to hold on to the wire grid of the cage with its four paws. The score was "1" if the mouse was able to hold on to the wire grid of the cage with its four paws but shortly fell off the grid. The score was "2" if the mouse was able to grab the wire grid of the cage with its two fore paws but was not able to hold on to the wire grid of the cage with its four paws. The score was "3" if the mouse was not able to grab the wire grid with its paws. For the limb reflexes, the number of the limbs with abnormal reflexes or tremor was used as a score. The results were presented as score charts for individual animals over time for each of the tremor, the grip strength, and the limb reflexes (Fig. 9).

As shown in Fig. 9, at least two of the motor dysfunctions suggesting ALS, i.e., the tremor, reduction in grip strength and abnormal reflexes, were found in all mice of between 33 and 78 weeks of age.

Thus, the mutant mice show abnormalities similar to those of ALS and are thus useful as animal models of ALS.

### (6) Measurement of electromyographic activity of mutant TDP-43 knock-in mice

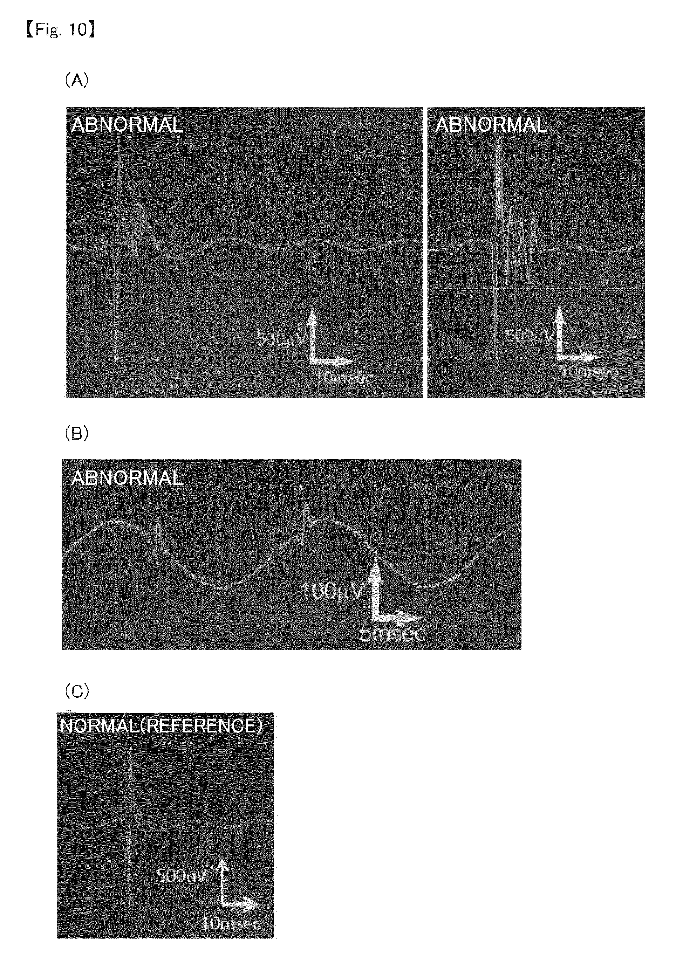

Electromyographic activity of mutant TDP-43 (G348C) knock-in mice at 4 months of age was measured. The results are given in Fig. 10.

In the mutant knock-in mouse, the duration before the motor unit potential (MUP) returned to baseline was prolonged abnormally (Fig. 10A), or weak, abnormal waves presumably due to fibrillation potential resulting from defects of the lower motor neurons appeared (Fig. 10B). For comparison, an electromyogram in which MUP duration was within a normal range is shown in Fig. 10C.

Thus, motor neuron dysfunction was reflected to the waveforms of the electromyogram in the mutant TDP-43 (G348C) knock-in mice.

### (7) Analysis of brain cells of mutant TDP-43 knock-in mice

After mutant TDP-43 knock-in mice at 5 months of age were subjected to perfusion-fixation with 4% formaldehyde, their brains were removed and fixed overnight again in 4% formaldehyde, which was replaced by sucrose. Subsequently, frozen sections were made at a thickness of 30 micrometers using Retoratome.

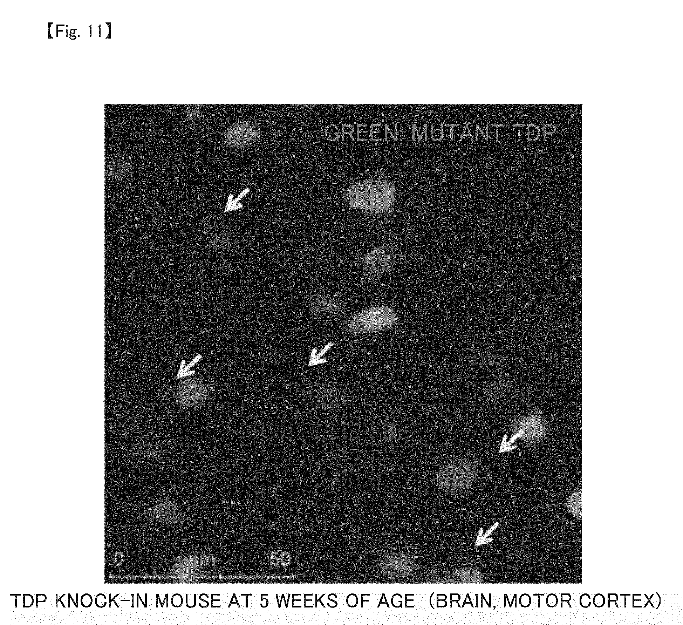

Fluorescence of Venus was observed on the sections under a fluorescence microscope. As shown in Fig. 11, the mutant TDP-43 formed cytoplasmic inclusions in addition to the inclusions localized to the nucleus.

Thus, the mutant TDP-43 knock-in mice are useful as an animal model of ALS which shows symptoms similar to those of human ALS as well as an animal model of FTLD which shows symptoms similar to those of human FTLD.

### (8) Analysis of higher brain function of mutant TDP-43 knock-in mice

Higher brain function of mutant TDP-43 knock-in mice from 5 months to 9 months of age was analyzed as described below.

### == Home cage activity test ==

First, a home cage activity test was performed using the mutant TDP-43 (A382T) knock-in mice to evaluate environmental adaptability of the mice.

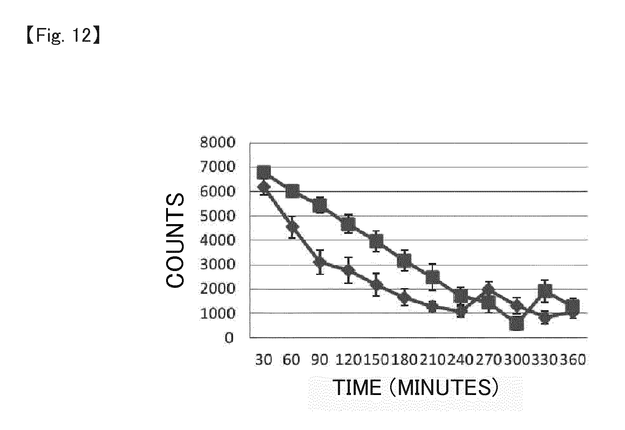

For 12 mutant TDP-43 (A382T) knock-in mice and 11 wild-type mice, horizontal activity was measured using a behavioral analysis system (Melquest, Ltd., SCANET) from immediately after each mouse was returned to its home cage to 360 minutes later. The number of times (counts) the infrared beam in the device is interrupted by the mouse was taken every 30 minutes. The result is shown in Fig. 12 (solid square: mutant mice; solid circle: wild-type mice).

As can be seen from the graph in Fig. 12, the mutant TDP-43 (A382T) knock-in mice showed significantly higher locomotor activity than the wild-types (p = 0.0083) for 240 minutes from when the animal was returned to the cage. After that, however, no significant difference was found in locomotor activity of these mice. Thus, the mutant TDP-43 (A382T) knock-in mice adapt more slowly to environment than the wild-types.

### == Open field test ==

Next, an open field test was performed to evaluate resistance to anxiety of the mutant TDP-43 knock-in mice in an unknown environment.

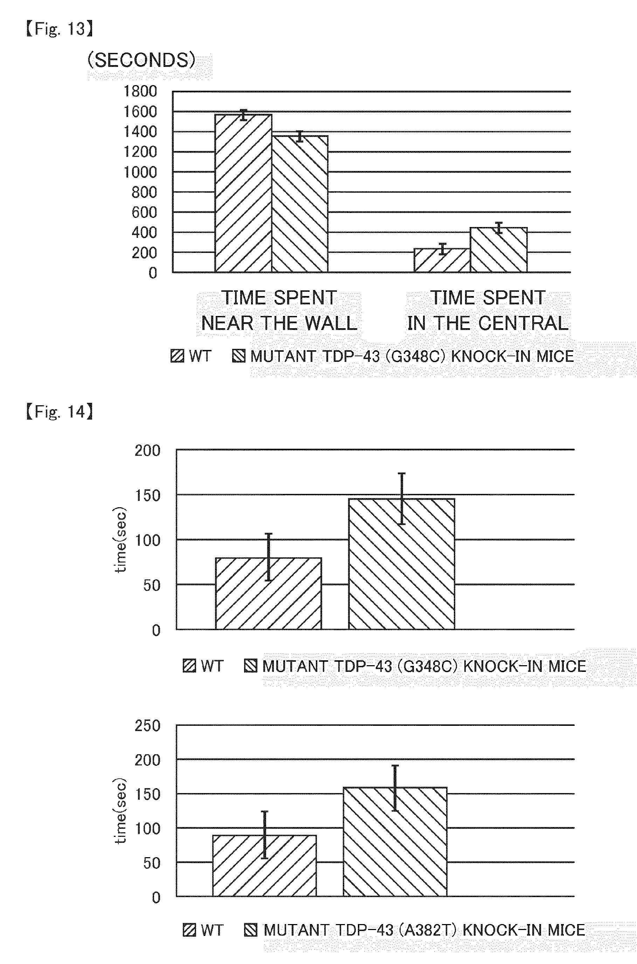

Mice were placed in a field of 50 cm x 50 cm. The time spent near the wall and the time spent in the central zone (18 cm x 18 cm) of the field were measured. For this test, 11 mutant TDP-43 (G348C) knock-in mice and 11 wild-type mice were used. The result is shown in Fig. 13 (left bars: wild-type mice; right bars: mutant mice).

As can be seen from the graph in Fig. 13, the mutant TDP-43 (G348C) knock-in mice spent significantly more time in the central zone than the wild-types did. Thus, the mutant TDP-43 (G348C) knock-in mice tend to be less anxious and feel less anxiety in unknown environments. The open field test was also performed with the mutant TDP-43 (A382T) knock-in mice and a similar tendency was observed.

### == Novel object recognition test ==

Next, a novel object recognition test was performed to evaluate resistance to anxiety to a novel object in the mutant TDP-43 knock-in mice.

Mice were placed in a chamber containing a novel object, and the time spent near the object was measured. For this test, 11 mutant TDP-43 (G348C) knock-in mice, 12 mutant TDP-43 (A348T) knock-in mice, and 11 wild-type mice were used. The result is shown in Fig. 14 (left bars: wild-type mice; right bars: mutant mice).

As can be seen from the graph in Fig. 14, the mutant TDP-43 (G348C) knock-in mice spent significantly more time near the novel object as compared to the wild-type mice (p = 0.1921). In addition, the mutant TDP-43 (A348T) knock-in mice also spent significantly more time near the novel object as compared to the wild-type mice (p = 0.1728). Thus, both types of the mutant TDP-43 (G348C) and TDP-43 (A348T) knock-in mice are less anxious and feel less anxiety to a novel object as compared to the wild-types.

### == Three-chamber test ==

Next, a three-chamber test was performed to eveluate sociability of the mutant TDP-43 knock-in mice.

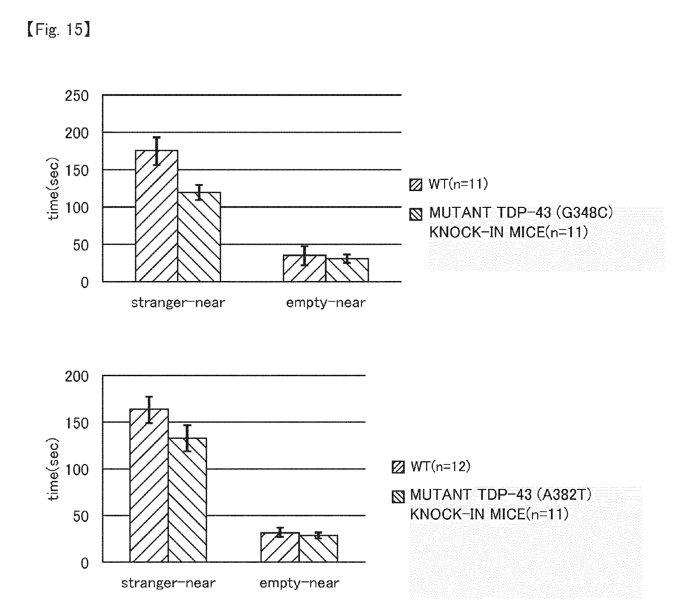

A cage with free access to three chambers was prepared and a mouse that had never lived with the subject before was placed in one of the three chambers. The time that the subject in the device spent in that chamber (stranger-near) and the time that the subject spent in one of the other chambers (empty-near) were measured. For this test, 11 mutant TDP-43 (G348C) knock-in mice, 11 mutant TDP-43 (A348T) knock-in mice, and 11 or 12 wild-type mice were used as the subject. The result is shown in Fig. 15 (left bars: wild-type mice; right bars: mutant mice).

As can be seen from the graph in Fig. 15, the mutant TDP-43 (G348C) knock-in mice spent significantly less time in the chamber with a stranger mouse as compared to the wild-type mice (p = 0.0198). The mutant TDP-43 (A348T) knock-in mice were similarly evaluated.. They tended to spend less time in the chamber with a mouse that the subject had never lived with before as compared to the wild-type mice. This indicated that the mutant TDP-43 knock-in mice tend to show lower sociability than the wild-type mice.

### == Radial 8-arm maze test ==

Next, a radial 8-arm maze test was performed to evaluate spatial memory of the mutant TDP-43 knock-in mice.

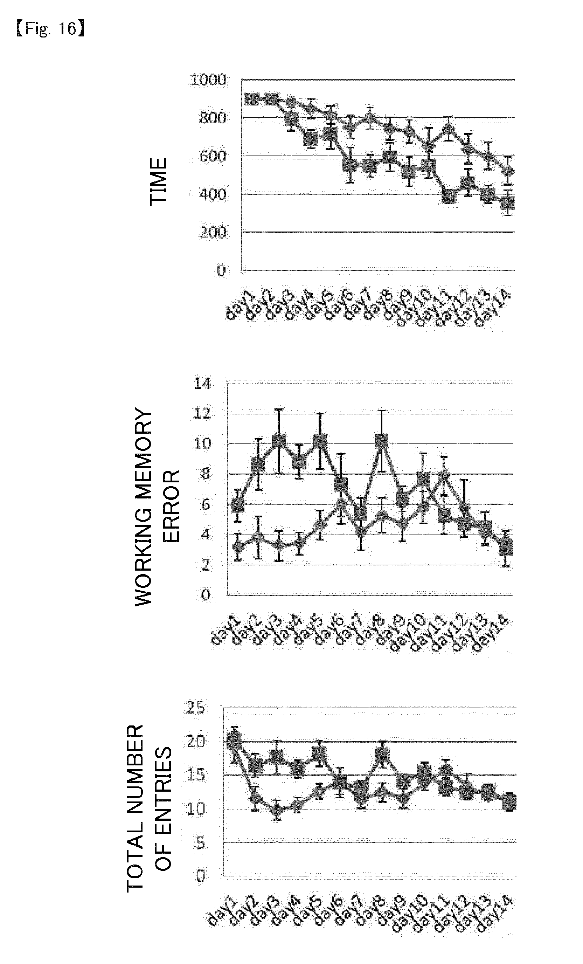

A device having eight arms radiating outward was prepared. Baits were placed at the ends of the respective arms and then a mouse was placed in the device. The time to consume all baits in the eight arms, the number of re-entries into a baited arm where the bait was already retrieved (the number of working memory errors), and the total number of entries into the arms were determined. This test was repeated for 14 days. For this test, 11 mutant TDP-43 (G348C) knock-in mice and 11 wild-type mice were used. The result is shown in Fig. 16 (solid square: mutant mice; solid circle: wild-type mice).

As can be seen from the graph in Fig. 16, the mutant TDP-43 (G348C) knock-in mice made more working memory errors as compared to the wild-type mice (p = 0.0039). In addition, the mutants needed less time to consume all baits in the eight arms as compared to the wild-type mice (P = 0.0027), but were overactive with many arm entries in total (p = 0.0077).

Thus, it was found that the mutant TDP-43 knock-in mice at or after 5 months of the age show higher brain dysfunction that is considered to reflect human FTLD, and are useful as animal models of FTLD.

### Industrial Applicability

The present invention enabled production of a mouse model having symptoms similar to human amyotrophic lateral sclerosis and/or human frontotemporal lobar degeneration.

## Claims

1. A non-human vertebrate wherein expression of a mutant protein with a substitution of tyrosine for alanine at a position corresponding to position 382 of SEQ ID NO. 1 or a mutant protein with a substitution of cysteine for glycine at a position corresponding to position 348 of SEQ ID NO. 1 is regulated by an endogenous TDP-43 gene promoter in at least one allele of an endogenous TDP-43 gene.

2. The vertebrate according to Claim 1, wherein the vertebrate has a mutation in which tyrosine is substituted for alanine at a position corresponding to position 382 of SEQ ID NO. 1 or a mutation in which cysteine is substituted for glycine at a position corresponding to position 348 of SEQ ID NO. 1, by an exogenous DNA, in at least one allele of the endogenous TDP-43 gene.

3. The vertebrate according to Claim 1 or 2, wherein the vertebrate is an animal model of amyotrophic lateral sclerosis.

4. The vertebrate according to Claim 1 or 2, wherein the vertebrate is an animal model of frontotemporal lobar degeneration.

5. The vertebrate according to any one of Claims 1 to 4, wherein the vertebrate is a knock-in mouse carrying a human TDP-43 gene.

6. A method of generating an animal model of amyotrophic lateral sclerosis, comprising the step of generating a mutation in a non-human vertebrate so that expression of a mutant protein with a substitution of tyrosine for alanine at a position corresponding to position 382 of SEQ ID NO. 1 or a mutant protein with a substitution of cysteine for glycine at a position corresponding to position 348 of SEQ ID NO. 1 is regulated by an endogenous TDP-43 gene promoter in at least one allele of an endogenous TDP-43 gene of the vertebrate.

7. The method according to Claim 6, wherein a mutation is generated in the vertebrate through the step of substituting tyrosine for alanine at a position corresponding to position 382 of SEQ ID NO. 1 or the step of substituting cysteine for glycine at a position corresponding to position 348 of SEQ ID NO. 1, by an exogenous DNA, in at least one allele of the endogenous TDP-43 gene of the vertebrate.

8. The method according to Claim 7, wherein the endogenous TDP-43 gene or a portion thereof, of the vertebrate is replaced with an exogenous human TDP-43 gene or a portion thereof.

9. The method of producing a mouse model of frontotemporal lobar degeneration, comprising the step of generating a mutation in a non-human vertebrate so that expression of a mutant protein with a substitution of tyrosine for alanine at a position corresponding to position 382 of SEQ ID NO. 1 or a mutant protein with a substitution of cysteine for glycine at a position corresponding to position 348 of SEQ ID NO. 1 is regulated by an endogenous TDP-43 gene promoter in at least one allele of the endogenous TDP-43 gene of the vertebrate.

10. The method according to Claim 9, wherein a mutation is generated in the vertebrate by means of substituting tyrosine for alanine at a position corresponding to position 382 of SEQ ID NO. 1 or substituting cysteine for glycine at a position corresponding to position 348 of SEQ ID NO. 1, by an exogenous DNA, in at least one allele of the endogenous TDP-43 gene of the vertebrate.

11. The method according to Claim 10, wherein the endogenous TDP-43 gene or a portion thereof, of the vertebrate is replaced with an exogenous human TDP-43 gene or a portion thereof.
